# EUROPÄISCHE PATENTSCHRIFT

(11) **EP 3 121 180 B1**
(45) Veröffentlichungstag und Bekanntmachung des Hinweises auf die Patenterteilung: **12.09.2018**
(21) Anmeldenummer: 16180044.6
(22) Anmeldetag: 19.07.2016
(51) Int. Cl.: B01J 31/22, B01J 31/24, C07C 67/38, C07F 17/02, C07F 9/572, C07F 9/655, C07F 9/6553, C07F 15/00, C07F 9/58, C07F 9/6506

(54) **BUTYL-VERBRÜCKTE DIPHOSPHINLIGANDEN FÜR DIE ALKOXYCARBONYLIERUNG**
BUTYL-BRIDGED DIPHOSPHINE LIGANDS FOR ALKOXYCARBONYLATION
LIGANDS DE DISPHOSPHINE RÉCULÉS PAR BUTYL POUR L'ALKOXY-CARBONYLATION

(30) Priorität: 23.07.2015 DE 102015213918
(43) Veröffentlichungstag der Anmeldung: 25.01.2017
(73) Patentinhaber: Evonik Degussa GmbH, 45128 Essen (DE)
(72) Erfinder: DONG, Kaiwu, 236800 Bo Zhou (CN); FANG, Xiangjie, Shanghai (CN); NEUMANN, Helfried, 18055 Rostock (DE); JACKSTELL, Ralf, 27478 Cuxhaven Altenwalde (DE); BELLER, Matthias, 18211 Ostseebad Nienhagen (DE); FRANKE, Robert, 45772 Marl (DE); HESS, Dieter, 45770 Marl (DE); DYBALLA, Katrin Marie, 45657 Recklinghausen (DE); FRIDAG, Dirk, 45721 Haltern am See (DE); GEILEN, Frank, 45721 Haltern am See (DE)

(56) Entgegenhaltungen:
- US-A- 3 213 093
- NIFANT'EV I E ET AL: "Steric and electronic factors in the promoting activity of diphosphine ligands in cyclohexene hydrocarbomethoxylation catalyzed by palladium acetate", KINETICS AND CATALYSIS, KLUWER ACADEMIC PUBLISHERS, BO, Bd. 53, Nr. 4, 26. Juli 2012 (2012-07-26), Seiten 462-469, XP035090146, ISSN: 1608-3210, DOI: 10.1134/S0023158412040076

## Beschreibung

Die Erfindung betrifft butyl-verbrückte Diphosphinverbindungen, Metallkomplexe dieser Verbindungen und deren Verwendung für die Alkoxycarbonylierung.

Die Alkoxycarbonylierung ethylenisch ungesättigter Verbindungen ist ein Prozess mit steigender Bedeutung. Unter einer Alkoxycarbonylierung versteht man die Umsetzung ethylenisch ungesättigter Verbindungen (Olefinen) mit Kohlenmonoxid und Alkoholen in Gegenwart eines Metall-Ligand-Komplexes zu den entsprechenden Estern. Üblicherweise wird als Metall Palladium eingesetzt. Das folgende Schema zeigt die allgemeine Reaktionsgleichung einer Alkoxycarbonylierung:

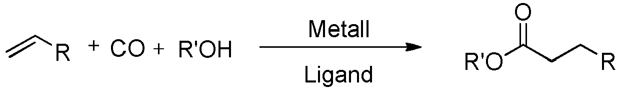

Unter den Alkoxycarbonylierungsreaktionen ist insbesondere die Reaktion von Ethen und Methanol zu 3-Methylpropionat (Ethen-Methoxycarbonylierung) von Bedeutung als Zwischenschritt für die Herstellung von Methylmethacrylat (S. G. Khokarale, E. J. Garcia-Suárez, J. Xiong, U. V. Mentzel, R. Fehrmann, A. Riisager, Catalysis Communications 2014, 44, 73-75). Die Ethen-Methoxycarbonylierung wird in Methanol als Lösemittel bei milden Bedingungen mit einem durch Phosphinliganden modifizierten Palladiumkatalysator durchgeführt.

Üblicherweise werden dabei zweizähnige Diphosphinverbindungen als Liganden eingesetzt. Ein sehr gutes katalytischen System wurde von Lucite - jetzt Mitsubishi Rayon - entwickelt und verwendet einen Liganden auf Basis von 1,2-Bis(di-tert-butylphosphinomethyl)benzol (DTBPMB) (W. Clegg, G. R. Eastham, M. R. J. Elsegood, R. P. Tooze, X. L. Wang, K. Whiston, Chem. Commun 1999, 1877-1878).

EP 0975574 A1 offenbart die Carbonylierung von 3-Methoxy-1-buten zu Methyl-3-pentenoat in Gegenwart von z.B. 1,4-bis(diphenylphosphino)-butan und 1,4-bis(dicyclohexylphosphino)-butan. Die Carbonylierung von langkettigen ethylenisch ungesättigten Verbindungen, wie z.B. Octen, wird nicht untersucht.

1,4-bis(dialkylphosphino)-butan-Verbindungen werden auch in anderen Bereichen als Liganden für Palladiumkatalysatoren eingesetzt. So offenbart die WO 02/10178 beispielsweise den Einsatz von 1,4-bis(di-adamantyl-phosphino)-butan als Ligand zur Veredelung von Halogenaromaten und zur Produktion von Arylolefinen, Dienen, Diarylen, Benzoesäure- und Acrylsäurederivaten, Arylalkanen und Aminen. Die Verwendung dieser Liganden für die Alkoxycarbonylierung ist jedoch nicht beschrieben.

In NIFANT'EV I. E. ET AL, "Steric and electronic factors in the promoting activity of diphosphine ligands in cyclohexene hydrocarbomethoxylation catalyzed by palladium acetate", KINETICS AND CATALYSIS (2012), 53, Nr. 4, Seiten 462-469 wird ein Verfahren zur Hydrocarbomethoxylierung von Cyclohexen beschrieben. Als Katalysator kommt ein Pd-Komplex mit Diphosphit-Liganden zum Einsatz.

Die vorliegende Erfindung hat die Aufgabe, neue Liganden für die Alkoxycarbonylierung bereitzustellen, mit denen sich gute Ausbeuten an Estern erzielen lassen. Insbesondere sollen die erfindungsgemäßen Liganden für die Alkoxycarbonylierung von langkettigen ethylenisch ungesättigten Verbindungen, beispielsweise C₈-Olefinen, und von Gemischen ethylenisch ungesättigter Verbindungen geeignet sein.

Diese Aufgabe wird gelöst durch butyl-verbrückte Diphosphinverbindungen, die an wenigstens einem Phosphoratom mit wenigstens einem Heteroarylrest substituiert sind. Diese Verbindungen eignen sich in besonderer Weise als zweizähnige Liganden für Palladiumkomplexe und führen zu erhöhten Ausbeuten bei der Alkoxycarbonylierung ethylenisch ungesättigter Verbindungen, insbesondere von C₈-Olefinen.

Die erfindungsgemäßen Diphosphinverbindungen sind Verbindungen gemäß Formel (**I**) 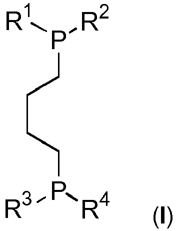 wobei
die Reste R¹ und R³ jeweils für einen -(C₃-C₂₀)-Heteroarylrest stehen;
und R² und R⁴ jeweils für einen -(C₁-C₁₂)-Alkylrest stehen
   und
R¹, R², R³, R⁴, falls diese für -(C₁-C₁₂)-Alkyl oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl,-COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl,-(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Erläuterungen zum Begriff (C₁-C₁₂)-Alkyl gelten insbesondere auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl und -N-[(C₁-C₁₂)-Alkyl]₂.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Die (C₃-C₁₂)-Cycloalkylgruppen weisen vorzugsweise 3 bis 8, besonders bevorzugt 5 oder 6, Ringatome auf.

Geeignete (C₃-C₁₂)-Cycloalkylgruppen sind insbesondere Cyclopropyl, Cyclobutyl, Cyclopentyl, Cyclohexyl, Cycloheptyl, Cyclooctyl, Cyclododecyl, Cyclopentadecyl, Norbonyl, Adamantyl.

Die Erläuterungen zum Begriff (C₃-C₁₂)-Cycloalkyl gelten insbesondere auch für die Cycloalkylgruppen in -O-(C₃-C₁₂)-Cycloalkyl, -S-(C₃-C₁₂)-Cycloalkyl, -COO-(C₃-C₁₂)-Cycloalkyl, -CONH-(C₃-C₁₂)-Cycloalkyl, -CO-(C₃-C₁₂)-Cycloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf und sind ggf. mit aliphatischen Seitenketten substituiert. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N, N(=O), C(=O), S(=O). Eine (C₃-C₁₂)-Heterocycloalkylgruppe im Sinne dieser Erfindung ist somit auch Ethylenoxid.

Geeignete (C₃-C₁₂)-Heterocycloalkylgruppen sind insbesondere Tetrahydrothiophenyl, Tetrahydrofuryl, Tetrahydropyranyl und Dioxanyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest, Furyl ist ein C₅-Heteroarylrest.

Geeignete (C₃-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Der Begriff Halogen umfasst insbesondere Fluor, Chlor, Brom und lod. Besonders bevorzugt sind Fluor und Chlor.

In einer Ausführungsform können die Reste R¹, R², R³, R⁴, falls diese für -(C₁-C₁₂)-Alkyl oder -(C₃-C₂₀)-Heteroaryl stehen,
jeweils unabhängig voneinander mit einem oder mehreren Substituenten ausgewählt aus-(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C3-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H,-NH₂, Halogen substituiert sein.

In einer Ausführungsform können die Reste R¹, R², R³, R⁴, falls diese für -(C₁-C₁₂)-Alkyl oder -(C₃-C₂₀)-Heteroaryl stehen,
jeweils unabhängig voneinander mit einem oder mehreren Substituenten ausgewählt aus-(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl,-(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, substituiert sein.

In einer Ausführungsform können die Reste R¹, R², R³, R⁴, falls diese für -(C₁-C₁₂)-Alkyl oder -(C₃-C₂₀)-Heteroaryl stehen,
jeweils unabhängig voneinander mit einem oder mehreren Substituenten ausgewählt aus-(C₁-C₁₂)-Alkyl, -O-(C₁-C₁₂)-Alkyl-(C₆-C₂₀)-Aryl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl substituiert sein.

In einer Ausführungsform können die Reste R¹, R², R³, R⁴, falls diese für -(C₁-C₁₂)-Alkyl oder -(C₃-C₂₀)-Heteroaryl stehen,
jeweils unabhängig voneinander mit einem oder mehreren Substituenten ausgewählt aus-(C₁-C₁₂)-Alkyl und -(C₃-C₂₀)-Heteroaryl substituiert sein.

In einer Ausführungsform sind die Reste R¹, R², R³, R⁴ unsubstituiert, falls diese für -(C₁-C₁₂)-Alkyl oder -(C₃-C₂₀)-Heteroaryl stehen,

In einer Ausführungsform sind die Reste R¹, R³ jeweils unabhängig voneinander ausgewählt aus Heteroarylresten mit fünf bis zehn Ringatomen, vorzugsweise fünf oder sechs Ringatomen.

In einer Ausführungsform stellen die Reste R¹, R³ einen Heteroarylrest mit fünf Ringatomen dar.

In einer Ausführungsform sind die Reste R¹, R³ jeweils unabhängig voneinander ausgewählt aus Heteroarylresten mit sechs bis zehn Ringatomen.

In einer Ausführungsform stellen die Reste R¹, R³ einen Heteroarylrest mit sechs Ringatomen dar.

In einer Ausführungsform sind die Reste R¹, R³ ausgewählt aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl, wobei die genannten Heteroarylreste wie oben beschrieben substituiert sein können.

In einer Ausführungsform sind die Reste R¹, R³ ausgewählt aus Furyl, Thienyl, Pyrrolyl, Imidazolyl, Pyridyl, Pyrimidyl, Indolyl, wobei die genannten Heteroarylreste wie oben beschrieben substituiert sein können.

In einer Ausführungsform sind die Reste R¹, R³ ausgewählt aus 2-Furyl, 2-Thienyl, 2-Pyrrolyl, 2-Imidazolyl, 2-Pyridyl, 2-Pyrimidyl, 2-Indolyl, wobei die genannten Heteroarylreste wie oben beschrieben substituiert sein können.

In einer Ausführungsform sind die Reste R¹, R³ ausgewählt aus 2-Furyl, 2-Thienyl, N-Methyl-2-Pyrrolyl, N-Phenyl-2-Pyrrolyl, N-(2-Methoxyphenyl)-2-Pyrrolyl, 2-Pyrrolyl, N-Methyl-2-Imidazolyl, 2-Imidazolyl, 2-Pyridyl, 2-Pyrimidyl, N-Phenyl-2-Indolyl, 2-Indolyl, wobei die genannten Heteroarylreste nicht weiter substituiert sind.

Besonders bevorzugt stellen die Reste R¹, R³ Pyridyl dar, insbesondere 2-Pyridyl.

In einer Ausführungsform stellen R¹ und R³ einen Pyridylrest, bevorzugt 2-Pyridyl, dar und R² und R⁴ stehen für -(C₁-C₁₂)-Alkyl, wobei R¹, R², R³ und R⁴ jeweils wie oben beschrieben substituiert sein können.

In einer Ausführungsform handelt es sich bei der erfindungsgemäßen Diphosphinverbindungen um eine Verbindung gemäß Formel (**1**):

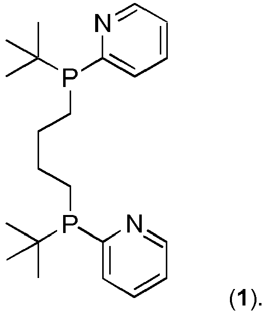

Die Erfindung betrifft weiterhin Komplexe umfassend Pd und eine erfindungsgemäße Diphosphinverbindung. Bei diesen Komplexen dient die erfindungsgemäße Diphosphinverbindung als zweizähniger Ligand für das Metallatom. Die Komplexe dienen beispielsweise als Katalysatoren für die Alkoxycarbonylierung. Mit den erfindungsgemäßen Komplexen lassen sich hohe Ausbeuten bei der Alkoxycarbonylierung einer Vielzahl unterschiedlicher ethylenisch ungesättigter Verbindungen erzielen.

Die erfindungsgemäßen Komplexe können außerdem weitere Liganden umfassen, die an das Metallatom koordinieren. Dabei handelt es sich beispielsweise um ethylenisch ungesättigte Verbindungen oder Anionen. Geeignete zusätzliche Liganden sind beispielsweise Styrol, Acetat-Anionen, Maleinimide (z.B. N-Methylmaleinimid), 1,4-Naphthochinon, Trifluoroacetat-Anionen oder Chloridanionen.

Die Erfindung betrifft weiterhin die Verwendung einer erfindungsgemäßen Diphosphinverbindung zur Katalyse einer Alkoxycarbonylierungsreaktion. Dabei kann die erfindungsgemäße Verbindung insbesondere als erfindungsgemäßer Metallkomplex eingesetzt werden.

Die Erfindung betrifft außerdem ein Verfahren umfassend die Verfahrensschritte:
a) Vorlegen einer ethylenisch ungesättigten Verbindung;
b) Zugabe einer erfindungsgemäßen Diphosphinverbindung und einer Verbindung, welche Pd umfasst,
   oder Zugabe eines erfindungsgemäßen Komplexes umfassend Pd und eine erfindungsgemäße Diphosphinverbindung;
c) Zugabe eines Alkohols;
d) Zuführen von CO;
e) Erwärmen des Reaktionsgemisches, wobei die ethylenisch ungesättigte Verbindung zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.
Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

In einer Ausführungsform umfasst die ethylenisch ungesättigte Verbindung 4 bis 30 Kohlenstoffatome, bevorzugt 6 bis 22 Kohlenstoffatome, besonders bevorzugt 8 bis 12 Kohlenstoffatome. In einer besonders bevorzugten Ausführungsform umfasst die ethylenisch ungesättigte Verbindung 8 Kohlenstoffatome.

Die ethylenisch ungesättigten Verbindungen können zusätzlich zu der einen oder mehreren Doppelbindungen weitere funktionelle Gruppen enthalten. Vorzugsweise umfasst die ethylenisch ungesättigte Verbindung eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten. Dabei umfasst die ethylenisch ungesättigte Verbindung vorzugsweise insgesamt 2 bis 30 Kohlenstoffatome, bevorzugt 2 bis 22 Kohlenstoffatome, besonders bevorzugt 2 bis 12 Kohlenstoffatome.

In einer Ausführungsform umfasst die ethylenisch ungesättigte Verbindung keine weiteren funktionellen Gruppen außer Kohlenstoff-Kohlenstoff-Doppelbindungen.

In einer besonders bevorzugten Ausführungsform handelt es sich bei der ethylenisch ungesättigten Verbindung um ein unfunktionalisiertes Alken mit mindestens einer Doppelbindung und 2 bis 30 Kohlenstoffatomen, bevorzugt 6 bis 22 Kohlenstoffatomen, weiter bevorzugt 8 bis 12 Kohlenstoffatomen, am meisten bevorzugt 8 Kohlenstoffatomen.

Geeignete ethylenisch ungesättigte Verbindungen sind beispielsweise:
Ethen;
Propen;
C4-Olefine, wie 1-Buten, cis-2-Buten, trans-2-Buten, Gemisch von cis- und trans-2-Buten, Isobuten, 1,3-Butadien; Raffinat I bis III, Crack-C4
C5-Olefine, wie 1-Penten, 2-Penten, 2-Methyl-1-buten, 2-Methyl-2-buten, 2-Methyl-1,3-butadien (Isopren), 1,3-Pentadien;
C6-Olefine, wie Tetramethylethylen, 1,3-Hexadien, 1,3-Cyclohexadien;
C7-Olefine, wie 1-Methylcyclohexen, 2,4-Heptadien, Norbonadien;
C8-Olefine, wie 1-Octen, 2-Octen, Cycloocten, Di-n-buten, Di-iso-buten, 1,5-Cyclooctadien, 1,7-Octadien;
C9-Olefine, wie Tripropen;
C10-Olefine, wie Dicylcopentadien;
Undecene;
Dodecene;
interne C14-Olefine;
interne C15- bis C18-Olefine;
lineare oder verzweigte, cyclische, acyclische oder teilweise cyclische, interne C15- bis C30-Olefine;
Triisobuten, Tri-n-buten;
Terpene, wie Limonen, Geraniol, Farnesol, Pinen, Myrcen, Carvon, 3-Caren;
mehrfach ungesättigte Verbindungen mit 18 Kohlenstoffatomen, wie Linolsäure oder Linolensäure;
Ester ungesättigter Carbonsäuren, wie Vinylester von Essig- oder Propansäure, Alkylester ungesättigter Carbonsäuren, Methyl- oder Ethylester von Acrylsäure oder Methacrylsäure, Ölsäureester, Ölsäure Methyl- oder Ethylester, Ester der Linol- oder Linolensäure; Vinylverbindungen, wie Vinylacetat, Vinylcyclohexen, Styrol, alpha-Methylstyrol, 2-Isopropenylnaphthalin;
2-Methyl-2-pentenal, Methyl-3-Pentenoat, Methacrylanhydrid.

In einer Variante des Verfahrens ist die ethylenisch ungesättigte Verbindung ausgewählt aus Propen, 1-Buten, cis- und/oder trans-2-Buten, oder Mischungen davon.

In einer Variante des Verfahrens ist die ethylenisch ungesättigte Verbindung ausgewählt aus 1-Penten, cis- und/oder trans-2-Penten, 2-Methyl-1-buten, 2-Methyl-2-buten, 3-Methyl-1-buten, oder Mischungen davon.

In einer bevorzugten Ausführungsform ist die ethylenisch ungesättigte Verbindung ausgewählt aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, n-Octen, 1-Octen, 2-Octen, oder Mischungen davon.

In einer Variante wird ein Gemisch ethylenisch ungesättigter Verbindungen eingesetzt. Als Gemisch wird im Sinne dieser Erfindung eine Zusammensetzung bezeichnet, die wenigsten zwei verschiedene ethylenisch ungesättigte Verbindungen enthält, wobei der Anteil jeder einzelnen ethylenisch ungesättigten Verbindungen vorzugsweise wenigstens 5 Gewichts-% bezogen auf das Gesamtgewicht des Gemisches beträgt.

Vorzugsweise wird ein Gemisch ethylenisch ungesättigter Verbindungen mit jeweils 2 bis 30 Kohlenstoffatomen, bevorzugt 4 bis 22 Kohlenstoffatomen, besonders bevorzugt 6 bis 12 Kohlenstoffatomen, am meisten bevorzugt 8 bis 10 Kohlenstoffatomen eingesetzt.

Geeignete Gemische ethylenisch ungesättigter Verbindungen sind die sogenannten Raffinate I bis III. Raffinat I umfasst 40 bis 50 % iso-Buten, 20 bis 30 % 1-Buten, 10 bis 20 % cis- und trans-2-Buten, bis zu 1 % 1,3-Butadien und 10 bis 20 % n-Butan und Isobutan. Das Raffinat II ist ein Teil der beim Naphthacracken entstehenden C₄-Fraktion und besteht im Wesentlichen aus den isomeren *n*-Butenen, Isobutan und *n*-Butan nach Abtrennung von Isobuten aus Raffinat I. Raffinat III ist ein Teil der beim Naphthacracken entstehenden C₄-Fraktion und besteht im Wesentlichen aus den isomeren *n*-Butenen und *n*-Butan.

Ein weiteres geeignetes Gemisch ist Di-n-Buten, auch bezeichnet als Dibuten, DNB oder DnB. Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder teilweise umgesetzt werden. Neben dem dimeren Di-n-Buten, entstehen in der Regel auch höhere Oligomere (Tributen C12, Tetrabuten C16), die nach der Reaktion destillativ abgetrennt werden. Diese können ebenfalls als Edukte eingesetzt werden.

In einer bevorzugten Variante wird ein Gemisch umfassend iso-Buten, 1-Buten, cis- und trans-2-Buten eingesetzt. Vorzugsweise umfasst das Gemisch 1-Buten, cis- und trans-2-Buten.

Die erfindungsgemäße Alkoxycarbonylierung wird durch den erfindungsgemäßen Pd-Komplex katalysiert. Der Pd-Komplex kann dabei in Verfahrensschritt b) entweder als präformierter Komplex umfassend Pd und den erfindungsgemäßen Phosphinliganden zugegeben werden, oder *in situ* aus einer Verbindung, welche Pd umfasst, und dem freien Phosphinliganden gebildet werden. Dabei wird die Verbindung, welche Pd umfasst, auch als Katalysatorvorstufe bezeichnet.

In dem Fall, dass der Katalysator *in situ* gebildet wird, kann der Ligand im Überschuss zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂].

Vorzugsweise handelt es sich bei der Verbindung, welche Pd umfasst, um PdCl₂, Pd(acac)₂ oder Pd(OAc)₂. Besonders geeignet ist PdCl₂.

Der Alkohol im Verfahrensschritt c) kann verzweigt oder linear sein, cyclisch, alicyclisch, teilweise zyklisch oder aliphatisch und stellt insbesondere ein C₁- bis C₃₀-Alkanol dar. Es können Monoalkohole oder Polyalkohole verwendet werden.

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Es kann sich dabei um einen Monoalkohol oder einen Polyalkohol handeln.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl- , Amino-, Ether-, Thioether-, Aryl-, Heteroaryl- oder Silylgruppen, und/oder Halogensubstituenten enthalten.

In einer Ausführungsform umfasst der Alkohol keine weiteren funktionellen Gruppen außer Hydroxylgruppen.

Der Alkohol kann ungesättigte und aromatische Gruppen enthalten. Vorzugsweise handelt es sich jedoch um einen aliphatischen Alkohol.

Als aliphatischer Alkohol wird im Rahmen dieser Erfindung ein Alkohol bezeichnet, der keine aromatischen Gruppen umfasst, also beispielsweise ein Alkanol, Alkenol oder Alkinol. Somit sind auch ungesättigte, nicht-aromatische Alkohole zugelassen.

In einer Ausführungsform handelt es sich bei dem Alkohol um ein Alkanol mit einer oder mehrerer Hydroxylgruppen und 1 bis 30 Kohlenstoffatomen, bevorzugt 1 bis 22 Kohlenstoffatomen, besonders bevorzugt 1 bis 12 Kohlenstoffatomen, am meisten bevorzugt 1 bis 6 Kohlenstoffatomen.

In einer Variante des Verfahrens ist der Alkohol im Verfahrensschritt c) ausgewählt aus der Gruppe der Monoalkohole.

In einer Variante des Verfahrens ist der Alkohol im Verfahrensschritt c) ausgewählt aus: Methanol, Ethanol, 1-Propanol, Iso-Propanol, Iso-Butanol, tert-Butanol, 1-Butanol, 2-Butanol, 1-Pentanol, 2-Pentanol, 3-Pentanol, 1-Hexanol, Cyclohexanol, Phenol, 2-Ethylhexanol, Isononanol, 2-Propylheptanol.

In einer bevorzugten Variante ist der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol im Verfahrensschritt c) ausgewählt aus der Gruppe der Polyalkohole.

In einer Variante des Verfahrens ist der Alkohol im Verfahrensschritt c) ausgewählt aus: Diolen, Triolen, Tetraolen.

In einer Variante des Verfahrens ist der Alkohol im Verfahrensschritt c) ausgewählt aus: Cyclohexan-1,2-diol, 1,2-Ethandiol, 1,3-Propandiol, Glycerol, 1,2,4-Butantriol,2-Hydroxymethyl-1,3-Propandiol, 1,2,6-Trihydroxyhexan, Pentaerythritol, 1,1,1-Tri(hydroxymethyl)ethan, Brenzkatechin, Resorcin und Hydroxyhydrochinon.

In einer Variante des Verfahrens ist der Alkohol im Verfahrensschritt c) ausgewählt aus: Sucrose, Fructose, Mannose, Sorbose, Galactose und Glucose.

In einer bevorzugten Variante des Verfahrens ist der Alkohol im Verfahrensschritt c) ausgewählt aus Methanol, Ethanol, 1-Propanol, 1-Butanol, 1-Pentanol, 1-Hexanol.

In einer besonders bevorzugten Variante des Verfahrens ist der Alkohol im Verfahrensschritt c) ausgewählt aus: Methanol, Ethanol.

In einer besonders bevorzugten Variante des Verfahrens ist der Alkohol im Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, ausgewählt aus: Toluol, Xylol, Tetrahydrofuran (THF) oder Methylenchlorid (CH₂Cl₂).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um die ethylenisch ungesättigte Verbindung zu einem Ester umzusetzen.

Das molare Verhältnis von der in Schritt a) vorgelegten ethylenisch ungesättigten Verbindung zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu der in Schritt a) vorgelegten ethylenisch ungesättigten Verbindung beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis der erfindungsgemäßen Diphosphinverbindung zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

Vorzugsweise wird das Verfahren unter Zusatz einer Säure durchgeführt. In einer Variante umfasst das Verfahren deshalb zusätzlich den Schritt c'): Zugabe einer Säure zum Reaktionsgemisch. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ ≤ 3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt c') zugesetzter Säure 0,3 bis 40 mol-%, bevorzugt 0,4 bis 15 mol-%, besonders bevorzugt 0,5 bis 5 mol-%, am meisten bevorzugt 0,6 bis 3 mol-%, bezogen auf die Stoffmenge der in Schritt a) eingesetzten ethylenisch ungesättigten Verbindung.

### Beispiele

Die folgenden Beispiele veranschaulichen die Erfindung.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, und Pierre Granger, Pure Appl. Chem., 2001, 73, 1795-1818; Robin K. Harris, Edwin D. Becker, Sonia M. Cabral de Menezes, Pierre Granger, Roy E. Hoffman und Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Darstellung von Chlor-2-pyridyl-tert-butylphosphin (Vorstufe A)

Der Grignard für die Synthese von Chlor-2-pyridyl-t-butylphosphin wird nach der "Knochelmethode" mit Isopropylmagnesiumchlorid dargestellt (Angew. Chem. 2004, 43, 2222-2226). Die Aufarbeitung erfolgt nach der Methode von Budzelaar (Organometallics 1990, 9, 1222-1227).

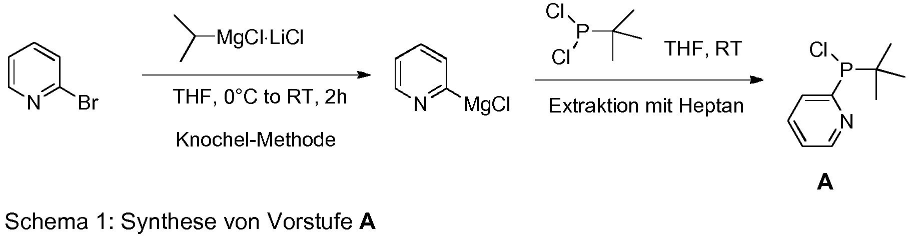

In einen 50 ml-Rundkolben mit Magnetrührer und Septum werden unter Argon 8,07 ml einer 1,3 M Isopropylmagnesium-chloridlösung (Knochel-Reagenz) gegeben und auf-15 °C gekühlt. Danach werden zügig 953.5 µl (10 mmol) 2-Bromopyridin zugetropft. Die Lösung wird sofort gelb. Man lässt auf -10 °C aufwärmen. Der Umsatz der Reaktion wird wie folgt bestimmt: man entnimmt ca. 100 µl Lösung und gibt sie in 1 ml einer gesättigten Ammoniumchloridlösung. Wenn die Lösung "sprudelt", dann hat sich noch nicht viel Grignard gebildet. Die wässrige Lösung wird mit einer Pipette Ether extrahiert und die organische Phase über Na₂SO₄ getrocknet. Von der etherischen Lösung wird ein GC aufgenommen.

Wenn sich viel Pyridin gebildet hat im Vergleich zu 2-Bromopyridin, dann hat man hohe Umsätze. Bei -10 °C hat sich wenig umgesetzt. Nach Aufwärmen auf Raumtemperatur und 1-2 stündigen Rühren wird die Reaktionslösung braungelb. Ein GC-Test zeigt vollständigen Umsatz. Jetzt kann die Grignardlösung zu einer Lösung von 1,748 g (11 mmol) Dichloro-tert-butylphosphin in 10 ml THF, die vorher auf -15 °C gekühlt worden ist, mit einer Spritzenpumpe langsam zugetropft werden. Wichtig ist, dass die Dichloro-tert-butylphosphinlösung gekühlt wird. Bei Raumtemperatur würde man erhebliche Mengen an Dipyridyl-tert-butylphosphin erhalten. Es entsteht anfangs eine klare gelbe Lösung, die dann trübe wird. Man lässt auf Raumtemperatur aufwärmen und über Nacht Rühren. Laut GCMS hat sich viel Produkt gebildet. Man entfernt das Lösungsmittel im Hochvakuum und erhält einen weißlichen Feststoff, der braune Stellen enthält. Der Feststoff wird mit 20 ml Heptan suspendiert und der Feststoff wird im Ultraschallbad zerkleinert. Nach dem Absetzen lassen des weißen Feststoffes wird die Lösung dekantiert. Der Vorgang wird zwei Mal mit je 10-20 ml Heptan wiederholt. Nach dem Einengen der Heptanlösung im Hochvakuum wird diese unter Vakuum destilliert. Bei 4,6 mbar, 120 °C Ölbad und 98 °C Übergangstemperatur kann das Produkt destilliert werden. Man erhält 1,08 g eines farblosen Öls. (50%).
Analytische Daten: ¹H NMR (300 MHz, C₆D₆): δ 8,36 (m, 1H, Py), 7,67 (m, 1H, Py), 7,03-6,93 (m, 1H, Py), 6,55-6,46 (m, 1H, Py), 1,07 (d, J = 13,3 Hz, 9H, t-Bu).
¹³C NMR (75 MHz, C₆D₆): δ 162,9, 162,6, 148,8, 135,5, 125,8, 125,7, 122,8, 35,3, 34,8, 25,9 und 25,8.
³¹P NMR (121 MHz, C₆D₆) δ 97,9.
MS (EI) *m*:*z* (relative Intensität) 201 (M⁺,2), 147(32), 145 (100), 109 (17), 78 (8), 57,1 (17).

### Herstellung von Verbindung 1

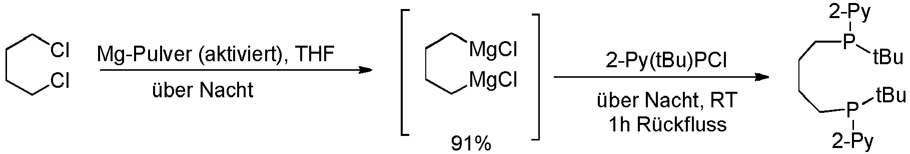

### (Lit: Graham Eastham et al., Patent US 6335471)

675 mg (27,8 mmol, 4 äquivalente) Mg-Pulver werden in der Glovebox in einen 250 ml-Rundkolben mit Stickstoffhahn und Magnetrührkern eingewogen und mit einem Septum verschlossen. Man legt an den Rundkoben Hochvakuum (ca 5x10⁻² mbar) an und erwärmt ihn auf 90 °C für 45 Minuten. Nach Abkühlen auf Raumtemperatur werden 2 Körnchen lod zugegeben und in 20 ml THF gelöst. Man rührt die Suspension ca. 10 Minuten bis die gelbe Farbe des Iods verschwunden ist. Nach Absetzen des Magnesiumpulvers wird die trübe THF-Lösung dekantiert und das aktivierte Magnesiumpulver zweimal mit 1-2 ml THF gewaschen. Dann werden erneut 20 ml frisches THF zugegeben. Bei Raumtemperatur wird eine Lösung von 755,5 µl (6,9 mmol) 1,4-Dichlorbutan in 70 ml THF mit der Spritzenpumpe langsam zugetropft. Die THF-Lösung ist klar und leicht gelb. Am nächsten Tag liegt eine dunkelgraue aber klare Lösung vor, die über Celite filtriert wird. Eine Probe der Grignardlösung wird wie folgt gequencht und im GC untersucht:
300 µl Grignardlösung wird mit 1 ml einer gesättigten wäßrigen Lösung von NH₄Cl gequencht und mit Ether extrahiert. Nach Trocknen über Na₂SO₄ wird von der Etherlösung ein GC aufgenommen. Es ist kein 1,4-Dichlorobutan mehr detektierbar, aber das entstandene Butan kann im GC nicht beobachtet werden.

### Der Gehalt an Grignardverbindung wird wie folgt bestimmt:

1 ml Grignardlösung wird mit 3 ml 0,1 M HCl gequencht und die überschüssige Säure mit 0,1 M NaOH titriert. Als Indikator ist eine wässrige 0,04%ige Bromkresollösung geeignet. Farbumschlag geht von gelb nach blau. Es sind 1,70 mL an 0,1 M NaOH verbraucht worden. 3mL-1,70mL = 1,3mL, das entspricht 0.13 mmol Grignardverbindung. Da ein Digrignard vorliegt ist die Grignardlösung 0,065M. Das sind bezogen auf 90 mL Lösung 85% an Grignardlösung. Der Grignard kann jetzt mit dem Chlorphosphin umgesetzt werden: In einem 250mL Dreihalskolben mit Rückflusskühler, Rührfisch und Stickstoffhahn werden unter Argon 1.94 g (9.75 mmol, 2.5 eq) Chloro-2-pyridyl-t-butylphosphin (Vorstufe **A**) in 10 mL THF gelöst und auf -60 °C gekühlt. Dann werden 60 mL der oben bestimmten Grignardlösung (0.065M, 3.9 mmol) langsam bei dieser Temperatur mit der Spritzenpumpe zugetropft. Die Lösung bleibt zunächst klar und wird dann intensiv gelb. Man lässt über Nacht auf Raumtemperatur aufwärmen und man erhält eine klare gelbe Lösung. Zur Vervollständigung der Reaktion erhitzt man 2 Stunden unter Rückfluß. Nach Abkühlen gibt man 1 mL H₂O dazu und die Lösung entfärbt sich und ein weißer Niederschlag fällt aus.

Nach Entfernen von THF im Hochvakuum erhält man einen zähen, hellgelben Feststoff. Man gibt 15 mL Wasser und 20 mL Ether dazu und erhält zwei homogene klare Phasen, die sich gut trennen lassen. Die wäßrige Phase wird 2 Mal mit Ether extrahiert. Nach Trocknen der organischen Phase mit Na₂SO₄ wird der Ether im Hochvakuum entfernt und man erhält einen zähes fast farbloses Öl. Dieses wird in 4 mL MeOH unter Erwärmen auf dem Wasserbad gelöst und über Celite filtriert. Bei -28 °C erhält man über Nacht 660 mg Produkt in Form von weißen, klebrigen Kristallen. (44%)
¹H NMR (300 MHz, C₆D₆): δ 8.54 (m, 2H, py), 7.37 (m, 2H, py), 6.96 (m, 2H, Py), 6.58 (m, 2H, Py), 2.68 (m, 2H, CH₂), 1.74 (m, 4H, CH₂), 1.52 (m; 2H, CH₂), 1.03 (d, J = 11.5 Hz, 18H, tBu).
¹³C NMR (75 MHz, C₆D₆): δ 162.8, 162.5 (q), 149.9, 134.3, 134.1, 132.0, 131.5 und 122.4 (py), 29.4, 29.3, 29.1, 29.0, 20.7, 20.5 (CH₂), 28.1 und 27.9 (tBu).·
³¹P NMR (121 MHz, C₆D₆) δ 8.2.
HRMS (ESI) m/z⁺ berechnet für: C₂₂H₃₄N₂P₂ (M+H)⁺ 389.227; gefunden: 389.2273.
EA berechnet für: C₂₂H₃₄N₂P₂: C, 68.02; H, 8.82; N, 7.21; P,15.95. gefunden: C, 68.16; H, 8.97; N, 7.07; P,15.91.

### Darstellung von Bis(diadamantylphosphinbutanboranaddukt) (Vorstufe B)

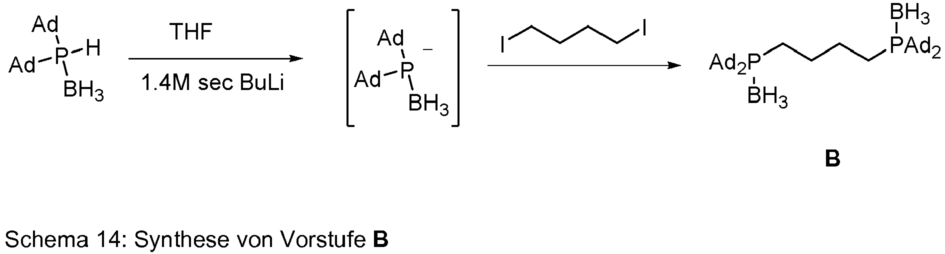

In einem 100 ml-Rundkolben mit Stickstoffhahn und Magentrührkern werden 214,7 mg (0,679 mmol) Diadamantylphosphinboranaddukt eingewogen. Man verschließt den Kolben mit einem Septum und gibt nach Sekurieren mit Argon 10 ml THF dazu. Das Boranaddukt ist gut löslich in THF und man erhält eine klare, farblose Lösung, die mit Trockeneis auf -78 °C gekühlt wird. Nach 15 Minuten Rühren wird 0,5 ml (0,70mmol) einer 1,4 M sec.-BuLi-Lösung langsam zugetropft. Nach dem Zutropfen erhält man eine leicht gelbliche, klare Lösung, die innerhalb von 3 Stunden auf Raumtemperatur gebracht wird. Man lässt die immer noch leicht gelbliche Lösung eine Stunde auf Raumtemperatur weiterühren und kühlt die Lösung wieder auf -78 °C. Zu dieser Lösung werden nun 42,6 µl (0,323mmol) Diiodbutan verdünnt mit 5 ml THF langsam zugetropft. Die gelbe Lösung entfärbt sich dabei. Man lässt über Nacht Aufwärmen und aus der Lösung fällt viel weißer Niederschlag aus. Man gibt 8 ml Wasser dazu und rührt 20 Minuten lang heftig. Weißer Feststoff schwimmt oben auf der Lösung. Die Lösung wird dekantiert und der weiße Feststoff wird dreimal mit MeOH gewaschen, um noch vorhandenes Wasser zu entfernen. Nach Trocken im Vakuum erhält man 210 mg (95%) Ausbeute eines weißen Feststoffes.
¹H NMR (300 MHz, CDCl₃): δ 2,11-1,89 (m, 36H, Ad), 1,79-1,68 (m, 24H, Ad), 1,67-1,49 (m, 8H, CH₂), 1,03-(-0,51) (m,breit),6H,BH₃).
¹³C NMR (75 MHz, CDCl₃): δ 37,8 und 36,6(Ad), 36,5 und 36,4(C), 28,1 und 28,0 (Ad), 27,9, 27,7, 15,1 und 14,7((CH₂)₄).
³¹P NMR (121 MHz, CDCl₃) δ 36,6-33,4 (m).

### Darstellung von Diadamantylphosphinboranaddukt (Vorstufe C)

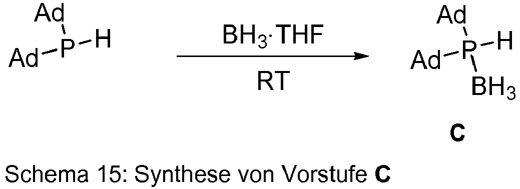

4,0 g (13,22 mmol) Diadamantylphosphin werden in einem 100 ml-Rundkolben mit Stickstoffhahn und ovalem Magentrührkern eingewogen, mit einem Septum verschlossen und sekuriert. Man suspendiert den Feststoff in 9 ml THF und gibt zu dieser Suspension 18,9 ml (18,9 mmol, 1 M) BH₃·THF Addukt zügig dazu. Die Suspension beginnt sich zunächst aufzulösen. Nach einiger Zeit fällt aber ein weißer Niederschlag aus. Man lässt über Nacht Rühren und entfernt das THF in Hochvakuum. Der weiße Rückstand wird unter Erwärmen (60 °C) auf dem Wasserbad in 250 ml Ethylacetat aufgenommen. Im warmen Ethylacetat ist das Boranaddukt gut löslich. Nach Zugabe von 6 Löffeln Kieselgel 60 (ca.12g) wird das Lösungsmittel am Rotationsverdampfer vollständig entfernt und das auf Kieselgel aufgezogene Produkt mit einer Combi-Flash Apparatur chromatograpiert. Als Laufmittel verwendet man 1:10 (Ethylacetat/Heptan). Man erhält 3,1 g (74%) an Diadamantylphosphinboranaddukt.
¹H NMR (300 MHz, CDCl₃): δ 3,71 (dq, 350,8 Hz und 6,6 Hz, 1H, PH), 2,01-1,94 (m, 18H, Ad), 1,74 (m, 12H, Ad), 1,05-(-0,35) (m,3H,BH₃).
¹³C NMR (75 MHz, CDCl₃): δ 37,9 und 36,4 (CH₂), 34,8 und 34,4 (C), 28,1 und 28,0 (CH).
³¹P NMR (121 MHz, CDCl₃) δ 42,8-40,0 (m).

### Herstellung von Ligand 2: Bis(diadamantylphosphino)butan (Vergleichsligand)

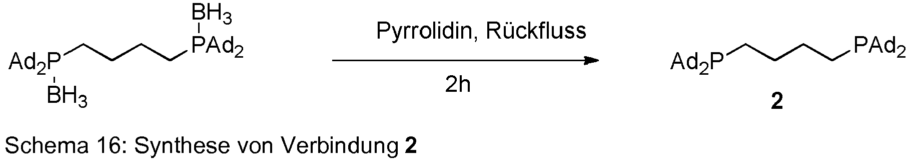

500 mg (0,728 mmol) Boranaddukt werden in einem 25 ml-Rundkolben mit Stickstoffhahn eingewogen und mit 10 ml absolutem Pyrrolidine versetzt. Die Suspension wird solange unter Rückfluß erhitzt, bis die Lösung farblos und klar ist (ca. 2 h). Nach Abkühlen wird das Pyrrolidin im Hochvakuum entfernt und man erhält einen weißen Rückstand. Dieser wird in 15 ml Toluol aufgenommen und auf 90 °C erwärmt. Die fast klare Lösung lässt sich schlecht filtrieren, da beim Abkühlen das Produkt wieder ausfällt. Aus dem Filtrat fällt im Kühlschrank (3 °C) ein weißer kristalliner Feststoff aus. Die Kristalle werden mit Toluol zweimal gewaschen und im Hochvakuum getrocknet. Man erhält 300 mg (62%) weiße Kristalle. Wegen der schlechten Löslichkeit bei Raumtemperatur wird ein1H, 13C und 31P- NMR in benzene-d6 bei 323 K aufgenommen.
¹H NMR (323 K, 400 MHz, C₆D₆): δ 2,12-1,92 (m, 16H, CH₂, Ad), 1,92-1,79 (m, 11H, CH₂, Ad), 1,75-1,64 (m, 16H, CH₂, Ad), 1,64-1,47 (m, 6H, CH₂, Ad), 1,45-1-23 (m, 18H, CH₂, Ad).
¹³C NMR (323 K, 100 MHz, C₆D₆): δ 41,5 und 41,4(Ad), 37,5 (Ad), 36,5 und 36,3 (C), 30,1 (CH₂), 29,3 und 29,2 (Ad, 17,5 und 17,3 (CH₂).
³¹P NMR (323 K, 162 MHz, C₆D₆) δ 25,71.

### Hochdruckexperimente

### Einsatzstoffe:

Di-n-Buten wurde auch wie folgt bezeichnet: Dibuten, DNB oder DnB.

Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder Teilweise umgesetzt werden. Neben dem dimeren Di-n-Buten, entstehen in der Regel auch höhere Oligomere (Tributen C12, Tetrabuten C16), die nach der Reaktion destillativ abgetrennt werden.

Ein industriell praktiziertes Verfahren zur Oligomerisierung von C4-Olefinen ist der sogenannte "OCTOL-Prozess".

Innerhalb der Patentliteratur beschreibt beispielsweise DE102008007081A1 eine auf dem OCTOL-Prozess beruhende Oligomerisierung. EP1029839A1 befasst sich mit der Fraktionierung der in dem OCTOL-Prozess entstehenden C8-Olefine.

Technisches Di-n-Buten besteht in der Regel zu 5 bis 30 % aus n-Octenen, 45 bis 75 % aus 3-Methylheptenen und zu 10 bis 35 % aus 3,4-Dimethylhexenen. Bevorzuge Ströme enthalten 10 bis 20 % n-Octene, 55 bis 65 % 3-Methylheptene und 15 bis 25 % 3,4-Dimethylhexene.

Para-Toluolsulfonsäure wurde wie folgt abgekürzt: pTSA, PTSA oder p-TSA. PTSA bezeichnet in diesem Text immer das para-Toluolsulfonsäure-Monohydrat.

### Allgemeine Vorschrift zur Durchführung der Hochdruckexperimente

### Allgemeine Versuchsvorschrift für Autoklavenversuche in Glasvials:

Es wird ein 300ml Parrreaktor verwendet. Diesem angepasst ist ein selbstgefertigter Aluminiumblock entsprechender Dimension welcher zu Heizen mittels handelsüblicher Magnetrührer z.B. der Firma Heidolph geeignet ist. Für das Innere des Autoklaven wurde eine runde Metallplatte der Stärke von ca. 1,5 cm angefertigt die 6 Bohrungen enthält, die dem Außendurchmesser der Glasvials entsprechen. Diesen Glasvials angepasst werden sie mit kleinen Magnetrührern bestückt. Diese Glasvials werden mit Schraubkappen und geeigneten Septen versehen und mit einer in der Glasbläserei angefertigten Spezialapparatur unter Argon mit den entsprechenden Reaktanden, Lösungsmittel und Katalysatoren und Additiven befüllt. Hierzu werden 6 Gefäße gleichzeitig befüllt, dies ermöglicht die Durchführung von 6 Reaktionen bei gleicher Temperatur und gleichem Druck in einem Experiment. Dann werden diese Glasgefäße mit Schraubkappen und Septen geschlossen und es wird jeweils ein kleine Spritzenkanüle geeigneter Größe durch die Septen gestochen. Dies ermöglicht später in der Reaktion den Gasaustausch. Diese Vials werden nun in der Metallplatte platziert und diese wird unter Argon in den Autoklaven überführt. Der Autoklav wird mit CO gespült und bei Raumtemperatur mit dem vorgesehenen CO-Druck befüllt. Dann wird mittels dem Magnetrührer unter Magnetrührung auf Reaktionstemperatur erhitzt und die Reaktion die entsprechende Zeit durchgeführt. Anschließend wird auf Raumtemperatur heruntergekühlt und der Druck langsam abgelassen. Anschließend wird der Autoklav mit Stickstoff gespült. Die Vials werden dem Autoklaven entnommen und mit einer definierten Menge eines geeigneten Standards versetzt. Es erfolgt eine GC Analyse mit deren Ergebnissen Ausbeuten und Selektivitäten bestimmt wurden.

### Analytik

GC Analytik Di-n-Buten: Für die GC Analyse wird ein Chromatograph der Firma Agilent GC Agilent 7890A mit einer 30 m HP5-Säule verwendet. Temperaturprofil: 35 °C, 10 min; 10 °C/min zu 200 °C; das Injektionsvolumen beträgt 1 µl mit einem Split von 50:1. Retentionszeiten für Di-n-Buten und Produkte: 10.784-13.502 min

Die aus dem Di-n-Buten gebildeten Ester werden im Folgenden als MINO bezeichnet (Methylisononanoat).

Retentionszeit für Etherprodukte unbekannter Isomerenverteilung: 15.312, 17.042, 17.244, 17.417 min

Retentionszeit für iso-C9-Ester 19.502-20.439 min (Hauptpeak: 19.990 min)

Retentionszeit für n-C9-Ester: 20.669, 20.730, 20.884, 21.266 min.

### Auswertung der Versuche

Für die Auswertung der katalytischen Experimente werden im Folgenden bestimmte Kennzahlen verwendet, die einen Vergleich der verschiedenen Katalysatorsysteme zulassen.

TON: Turnovernumber, definiert als mol Produkt pro mol Katalysatormetall ist ein Maß für die Produktivität des katalytischen Komplexes.

TOF: Turnoverfrequenz, definiert als TON pro Zeit für das Erreichen eines bestimmten Umsatzes, z.B. 50 %. Die TOF ist ein Maß für die Aktivität des katalytischen Systems.

Die im Folgenden angegebenen n-Selektivitäten beziehen sich auf den Anteil der endständigen Methoxycarbonylierung bezogen auf die Gesamtausbeute an Methoxycarbonylierungsprodukten.

Das n/iso-Verhältnis gibt das Verhältnis von endständig zu Estern umgesetzten Olefinen zu innenständig zu Estern umgesetzten Olefinen an.

### Methoxycarbonylierung von Di-n-Buten

Ligand **2** (Vergleichsbeispiel): Ein 25 ml-Schlenkgefäß wurde mit einer Stammlösung aus [Pd(acac)₂] (1,95 mg, 6.4 µmol), p-Toluolsulfonsäure (PTSA) (18,24 mg, 95.89 µmol) und MeOH (10 ml) befüllt. Ein 4 mL-Fläschchen wurde mit **2** (2,11 mg, 0,16 mol% bezogen auf die Stoffmenge von Di-n-Buten) befüllt und ein Magnetrührstab hinzugegeben. Danach wurden 1,25 ml der klaren gelben Stammlösung und Di-n-Buten (315 µl, 2 mmol) mit einer Spritze injiziert. Die molaren Anteile bezogen auf die Stoffmenge von Di-n-Buten betragen somit für Pd(acac)₂ 0.04 mol% und für PTSA 0.6 mol%. Das Fläschchen wurde auf einen Probenhalter gesetzt, der wiederum unter Argon-Atmosphäre in einen 300 ml-Parr-Autoklaven eingesetzt wurde. Nach dreimaligem Spülen des Autoklaven mit Stickstoff wurde der CO-Druck auf 40 bar eingestellt. Die Reaktion lief bei 120 °C 20 Stunden. Nach Abschluss der Reaktion wurde der Autoklav auf Raumtemperatur abgekühlt und vorsichtig entspannt. Isooctan wurde als interner GC-Standard zugesetzt. Ausbeute und Regioselektivität wurden mittels GC bestimmt. Keine MINO-Bildung wurde beobachtet.

Ligand **1:** Ein 25 ml-Schlenkgefäß wurde mit einer Stammlösung aus [Pd(acac)₂] (1,95 mg, 6.4 µmol), p-Toluolsulfonsäure (PTSA) (18,24 mg, 95.89 µmol) und MeOH (10 ml) befüllt. Ein 4 mL-Fläschchen wurde mit **1** (1,24 mg, 0,16 mol% bezogen auf die Stoffmenge von Di-n-Buten) befüllt und ein Magnetrührstab hinzugegeben. Danach wurden 1,25 ml der klaren gelben Stammlösung und Di-n-Buten (315 µl, 2 mmol) mit einer Spritze injiziert. Die molaren Anteile bezogen auf die Stoffmenge von Di-n-Buten betragen somit für Pd(acac)₂ 0.04 mol% und für PTSA 0.6 mol%. Das Fläschchen wurde auf einen Probenhalter gesetzt, der wiederum unter Argon-Atmosphäre in einen 300 ml-Parr-Autoklaven eingesetzt wurde. Nach dreimaligem Spülen des Autoklaven mit Stickstoff wurde der CO-Druck auf 40 bar eingestellt. Die Reaktion lief bei 120 °C 20 Stunden. Nach Abschluss der Reaktion wurde der Autoklav auf Raumtemperatur abgekühlt und vorsichtig entspannt. Isooctan wurde als interner GC-Standard zugesetzt. Ausbeute und Regioselektivität wurden mittels GC bestimmt. (Ausbeute MINO: 13 %, Regioselektivität n/iso: 59/41).

Dieser Versuch zeigt, dass der erfindungsgemäße Liganden **1** einen katalytisch aktiven Palladiumkomplex bildet, der die Alkoxycarbonylierung von Di-n-Buten katalysiert. Der strukturell ähnliche Ligand **2** ist hingegen nicht geeignet, die Alkoxycarbonylierung zu katalysieren.

## Patentansprüche

1. Verbindung gemäß Formel (**I**) 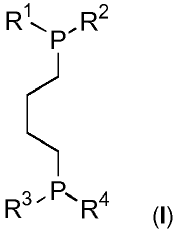 wobei
die Reste R¹ und R³ jeweils für einen -(C₃-C₂₀)-Heteroarylrest stehen;
und R² und R⁴ jeweils für einen -(C₁-C₁₂)-Alkylrest stehen
und
R¹, R², R³, R⁴, falls diese für -(C₁-C₁₂)-Alkyl oder -(C₃-C₂₀)-Heteroaryl stehen,
jeweils unabhängig voneinander mit einem oder mehreren Substituenten ausgewählt aus
-(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl,-(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

2. Verbindung nach Anspruch 1,
wobei R¹, R³ jeweils unabhängig voneinander ausgewählt sind aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

3. Verbindung nach einem der Ansprüche 1 oder 2,
gemäß der Formeln (**1**)

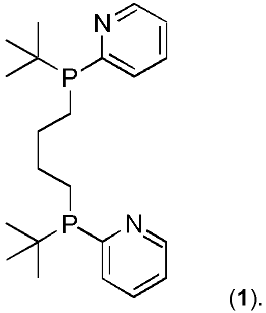

4. Komplex umfassend Pd und eine Verbindung nach einem der Ansprüche 1 bis 3.

5. Verfahren umfassend die Verfahrensschritte:
a) Vorlegen einer ethylenisch ungesättigten Verbindung;
b) Zugabe einer Verbindung nach einem der Ansprüche 1 bis 3 und einer Verbindung, welche Pd umfasst,
oder Zugabe eines Komplexes nach Anspruch 4;
c) Zugabe eines Alkohols;
d) Zuführen von CO;
e) Erwärmen des Reaktionsgemisches, wobei die ethylenisch ungesättigte Verbindung zu einem Ester umgesetzt wird.

6. Verfahren nach Anspruch 5,
wobei die ethylenisch ungesättigte Verbindung 2 bis 30 Kohlenstoffatom umfasst und optional eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten aufweist.

7. Verfahren nach einem der Ansprüche 5 bis 6,
wobei die ethylenisch ungesättigte Verbindung ausgewählt ist aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, di-n-Buten, oder Mischungen davon.

8. Verfahren nach einem der Ansprüche 5 bis 7,
wobei die ethylenisch ungesättigte Verbindung 6 bis 22 Kohlenstoffatome umfasst.

9. Verfahren nach einem der Ansprüche 5 bis 8,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

10. Verfahren nach einem der Ansprüche 5 bis 9,
wobei der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

11. Verwendung einer Verbindung gemäß einem der Ansprüche 1 bis 3 oder eines Komplexes gemäß Anspruch 4 zur Katalyse einer Alkoxycarbonylierungsreaktion.

## Claims

1. Compound of formula (**I**) 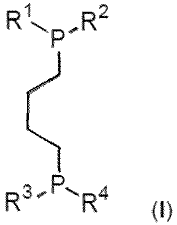 where
the R¹ and R³ radicals are each a -(C₃-C₂₀)-heteroaryl radical;
and R² and R⁴ are each a -(C₁-C₁₂)-alkyl radical; and
R¹, R², R³, R⁴, if they are -(C₁-C₁₂)-alkyl or -(C₃-C₂₀) -heteroaryl,
may each independently be substituted by one or more substituents selected from
-(C₁-C₁₂)-alkyl, -(C₃-C₁₂)-cycloalkyl, -(C₃-C₁₂)-heterocycloalkyl, -O-(C₁-C₁₂) -alkyl, -O-(C₁-C₁₂)-alkyl-(C₆-C₂₀)-aryl, -O-(C₃-C₁₂)-cycloalkyl, -S-(C₁-C₁₂)-alkyl, -S-(C₃-C₁₂)-cycloalkyl, -COO-(C₁-C₁₂)-alkyl, -COO-(C₃-C₁₂)-cycloalkyl, -CONH-(C₁-C₁₂)-alkyl, -CONH-(C₃-C₁₂)-cycloalkyl, -CO-(C₁-C₁₂)-alkyl, -CO-(C₃-C₁₂)-cycloalkyl, -N-[(C₁-C₁₂)-alkyl]₂, -(C₆-C₂₀)-aryl, -(C₆-C₂₀)-aryl-(C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl-(C₁-C₁₂)-alkyl, -(C₃-C₂₀)-heteroaryl-O-(C₁-C₁₂)-alkyl, -COOH, -OH, -SO₃H, -NH₂, halogen.

2. Compound according to Claim 1,
where R¹, R³ are each independently selected from furyl, thienyl, pyrrolyl, oxazolyl, isoxazolyl, thiazolyl, isothiazolyl, imidazolyl, pyrazolyl, furazanyl, tetrazolyl, pyridyl, pyridazinyl, pyrimidyl, pyrazinyl, benzofuranyl, indolyl, isoindolyl, benzimidazolyl, quinolyl, isoquinolyl.

3. Compound according to either of Claims 1 and 2, of the formula (**1**)

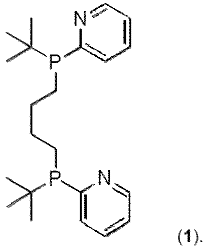

4. Complex comprising Pd and a compound according to any of Claims 1 to 3.

5. Process comprising the following process steps:
a) initially charging an ethylenically unsaturated compound;
b) adding a compound according to any of Claims 1 to 3 and a compound comprising Pd,
or adding a complex according to Claim 4;
c) adding an alcohol;
d) feeding in CO;
e) heating the reaction mixture, with conversion of the ethylenically unsaturated compound to an ester.

6. Process according to Claim 5,
wherein the ethylenically unsaturated compound comprises 2 to 30 carbon atoms and optionally one or more functional groups selected from carboxyl, thiocarboxyl, sulpho, sulphinyl, carboxylic anhydride, imide, carboxylic ester, sulphonic ester, carbamoyl, sulphamoyl, cyano, carbonyl, carbonothioyl, hydroxyl, sulphhydryl, amino, ether, thioether, aryl, heteroaryl or silyl groups and/or halogen substituents.

7. Process according to either of Claims 5 and 6,
wherein the ethylenically unsaturated compound is selected from ethene, propene, 1-butene, *cis-* and/or *trans*-2-butene, isobutene, 1,3-butadiene, 1-pentene, *cis-* and/or *trans*-2-pentene, 2-methyl-1-butene, 3-methyl-1-butene, 2-methyl-2-butene, hexene, tetramethylethylene, heptene, 1-octene, 2-octene, di-n-butene, and mixtures thereof.

8. Process according to any of Claims 5 to 7,
wherein the ethylenically unsaturated compound comprises 6 to 22 carbon atoms.

9. Process according to any of Claims 5 to 8,
wherein the compound comprising Pd in process step b) is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), palladium(cinnamyl) dichloride.

10. Process according to any of Claims 5 to 9,
wherein the alcohol in process step c) is selected from methanol, ethanol, 1-propanol, 1-butanol, 1-pentanol, 1-hexanol, 2-propanol, *tert*-butanol, 3-pentanol, cyclohexanol, phenol, and mixtures thereof.

11. Use of a compound according to any of Claims 1 to 3 or of a complex according to Claim 4 for catalysis of an alkoxycarbonylation reaction.

## Revendications

1. Composé selon la formule (I) 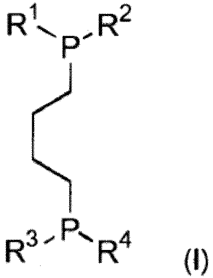 dans laquelle
les radicaux R¹ et R³ représentent chacun un radical hétéroaryle en (C₃-C₂₀) ;
et R² et R⁴ représentent chacun un radical alkyle en (C₁-C₁₂),
et
R¹, R², R³, R⁴, si ceux-ci représentent alkyle en (C₁-C₁₂) ou hétéroaryle en (C₃-C₂₀), peuvent être substitués chacun indépendamment les uns des autres par un ou plusieurs substituants choisis parmi :
alkyle en (C₁-C₁₂) , cycloalkyle en (C₃-C₁₂), hétérocycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂),-CONH-alkyle en (C₁-C₁₂), -CONH-cycloalkyle en (C₃-C₁₂),-CO-alkyle en (C₁-C₁₂), -CO-cycloalkyle en (C₃-C₁₂), -N-[alkyle en (C₁-C₁₂)]₂, aryle en (C₆-C₂₀), aryle en (C₆-C₂₀)-alkyle en (C₁-C₁₂), aryle en (C₆-C₂₀)-O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀) -O-alkyle en (C₁-C₁₂),-COOH, -OH, SO₃H, -NH₂, halogène.

2. Composé selon la revendication 1, dans lequel R¹, R³ sont chacun choisis indépendamment l'un de l'autre parmi furyle, thiényle, pyrrolyle, oxazolyle, isoxazolyle, thiazolyle, isothiazolyle, imidazolyle, pyrazolyle, furazanyle, tétrazolyle, pyridyle, pyridazinyle, pyrimidyle, pyrazinyle, benzofuranyle, indolyle, isoindolyle, benzimidazolyle, quinolyle, isoquinolyle.

3. Composé selon l'une quelconque des revendications 1 ou 2, selon la formule (1)

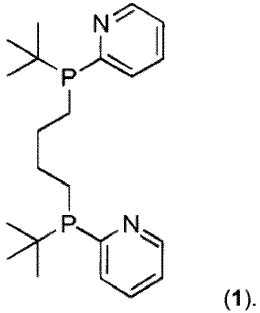

4. Complexe comprenant Pd et un composé selon l'une quelconque des revendications 1 à 3.

5. Procédé comprenant les étapes de procédé suivantes :
a) le chargement d'un composé éthyléniquement insaturé ;
b) l'ajout d'un composé selon l'une quelconque des revendications 1 à 3 et d'un composé qui comprend Pd, ou l'ajout d'un complexe selon la revendication 4 ;
c) l'ajout d'un alcool ;
d) l'introduction de CO ;
e) le chauffage du mélange réactionnel, le composé éthyléniquement insaturé étant transformé en un ester.

6. Procédé selon la revendication 5, dans lequel le composé éthyléniquement insaturé comprend 2 à 30 atomes de carbone et éventuellement un ou plusieurs groupes fonctionnels choisis parmi les groupes carboxyle, thiocarboxyle, sulfo, sulfinyle, anhydride d'acide carboxylique, imide, ester d'acide carboxylique, ester d'acide sulfonique, carbamoyle, sulfamoyle, cyano, carbonyle, carbonothioyle, hydroxyle, sulfhydryle, amino, éther, thioéther, aryle, hétéroaryle ou silyle et/ou les substituants halogène.

7. Procédé selon l'une quelconque des revendications 5 à 6, dans lequel le composé éthyléniquement insaturé est choisi parmi l'éthène, le propène, le 1-butène, le *cis-* et/ou le *trans*-2-butène, l'iso-butène, le 1,3-butadiène, le 1-pentène, le *cis-* et/ou le *trans*-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

8. Procédé selon l'une quelconque des revendications 5 à 7, dans lequel le composé éthyléniquement insaturé comprend 6 à 22 atomes de carbone.

9. Procédé selon l'une quelconque des revendications 5 à 8, dans lequel le composé qui comprend Pd à l'étape de procédé b) est choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le dichlorure de palladium(cinnamyle).

10. Procédé selon l'une quelconque des revendications 5 à 9, dans lequel l'alcool à l'étape de procédé c) est choisi parmi le méthanol, l'éthanol, le 1-propanol, le 1-butanol, le 1-pentanol, le 1-hexanol, le 2-propanol, le *tert*-butanol, le 3-pentanol, le cyclohexanol, le phénol ou leurs mélanges.

11. Utilisation d'un composé selon l'une quelconque des revendications 1 à 3 ou d'un complexe selon la revendication 4 pour la catalyse d'une réaction d'alcoxycarbonylation.
